# EUROPEAN PATENT SPECIFICATION

(11) **EP 0 929 308 B1**
(45) Date of publication and mention of the grant of the patent: **21.08.2002**
(21) Application number: 97928188.8
(22) Date of filing: 12.06.1997
(51) Int. Cl.: A61K 35/54, A61K 35/60, A61K 35/64, A61P 35/00

(54) **PHARMACEUTICAL COMPOSITION CONTAINING AN EMBRYONIC EXTRACT AS AN ACTIVE SUBSTANCE AND RELATIVE PREPARATION PROCESS**
PHARMAZEUTISCHE ZUSAMMENSETZUNG ENTHALTEND EINEN EMBRYONALEN EXTRAKT UND VERFAHREN ZU IHRER HERSTELLUNG
COMPOSITION PHARMACEUTIQUE CONTENANT UN EXTRAIT EMBRYONNAIRE, UTILISE COMME SUBSTANCE ACTIVE ET PROCEDE DE PREPARATION DE LADITE COMPOSITION

(30) Priority: 20.09.1996 IT MI961945
(43) Date of publication of application: 21.07.1999
(73) Proprietor: Carluccio, Adriana, 20129 Milano (IT); Biava, Pier Mario, 20123 Milano (IT)
(72) Inventor: Carluccio, Adriana, 20129 Milano (IT); Biava, Pier Mario, 20123 Milano (IT)
(74) Representative: Gervasi, Gemma, Dr.
(86) International application number: EP9703071
(87) International publication number: WO98011905

(56) References cited:
- DE-A- 4 400 640
- FR-A- 2 451 193
- BIAVA P M ET AL: "EFFECTS OF TREATMENT WITH EMBRYONIC AND UTERINE TISSUE HOMOGENATES ON LEWIS LUNG CARCINOMA DEVELOPMENT." CANCER LETT 41 (3). 1988. 265-270, XP002043907 cited in the application

## Description

### PRIOR ART

It is known that during pregnancy the cell differentiation takes to the formation of more and more specialized cells from undifferentiated and totipotent cells.

During the organogenesis period intervene, in fact, some regulating substances which prevent the unlimited multiplication of the cells, typical of the cancer development, characterizing them in a specific sense.

On the other hand the studies about the interactions between cancer cells and embryonic tissues show that the tumour development is reduced or suppressed during the period referring to the differentiation processes (Einhort, L. (1982) Oncodev. Biol. Med., 4, 219-229).

In fact the administration of tumorous agents before or during the organogenesis period produces malformations but not the induction of a tumour while, when the organogenesis is complete, the induction of tumours increases and the malformations rate decreases (Brent, R.L.) (1980) Radiation teratogenesis. Teratology, 21, 281-298).

According to these informations an experimentation has been carried out in order to determine the effects of embryonic homogenates on the growth and dissemination of the tumours in mice (Cancer Letters, 41 (1988) 265-270).

In said experimentation the effect of the administration to mice of homogenates deriving from mouse uteri and embryos at 9 days from coupling has been compared with the effect of the administration of homogenates derived from not gravid uteri and from normal liver.

The effect has been determined in mice wherein cells of Lewis pulmonary carcinoma had been subcutaneously implanted.

The formation of the primary tumour and the pulmonary metastases has been completely suppressed in the group of mice treated with gravid uteri homogenates, while the homogenates coming from not gravid uteri, from embryos and from normal liver showed no efficacy.

On the other hand the tumour represents a complex problem to the genesis thereof different factors contribute: oncogenes, lack of oncosuppressor genes, autocrine growth factors etc. A situation of such complexity can not be faced with therapeutic strategies based on the use of few substances and therefore the problem remaining open is to search for differentiated treatments dependent upon the various kinds of tumours.

### DETAILED DESCRIPTION OF THE INVENTION

Now we have found that extracts of embryos taken in specific stages of the embryonic development are effective in the treatment of the tumours and the other pathologies controlled by the p53 antioncogene and that said specific stages change dependent upon the species of origin of the extracts.

Therefore the present invention refers to pharmaceutical compositions containing as active substances extracts of embryos taken in specific stages of the embryonic development in mixture with pharmacologically acceptable diluents or excipients, suitable for the treatment of the tumours and the other pathologies controlled by the p53 antioncogene.

The present invention further refers to the process for the preparation of said extracts and said compositions.

Embryos suitable for the use according to the present invention are taken from the species comprising brachydanio rerio, trout, and drosophila.

The embryos are taken at specific stages of the embryonic development, different for the different pathologies and different for different species.

We have particularly found that the primitive tumour of the liver is successfully treated with trout embryonic extracts at the development stage of 20 somites; the glioblastoma multiforme is successfully treated with trout embryonic extracts at the development stage of 5 and 20 somites and with brachydanio rerio embryos at the medioblastula-gastrula stage; the ovarioncus is successfully treated with brachydanio rerio embryonic extracts at the development stage of medioblastula-gastrula; the Lewis tumour is successfully treated with drosophila embryonic extracts at the blastoderma stage.

The preparation of the extracts is carried out in a different way according to the kind of embryo and to the embryogenesis map elaborated for the species under examination, acting according to the methods described in detail in the below reported examples.

The preparation of the extracts is carried out by the following steps:
a) embryos at a stage specific of the embryonic development are taken;
b) said embryos are treated with several sonication cycles;
c) a solvent is added;
d) the mixture obtained in the step c) is stirred by a turbodiffuser;
e) the mixture is first filtered by 90 micrometers filters and then with 5-10 micrometers filters.

The sonication time of the step a) is preferally ranging from 1 to 4 hours. The solvent used in the step c) consists of a mixture of glycerin and a 30% v/v ethyl alcohol aqueous solution, in a 85:15 v/v ratio.

The ratio by weight between said solvent and said embryos is preferably ranging from 20:1 to 2:1.

The stirring of the step d) is preferably carried out at room temperature, as the other operations too, for a time from 2 hours to 15 hours.

The extracts according to the present invention have been used in an in vitro experimentation in order to determine their anti-tumour efficacy.

Such experimentation has been carried out by the following criteria:
1) it has been carried out on cultures in stable line of cells coming from tumours of various kind.
2) The cancer cells cultures have been treated with embryonic extracts represented by the medioblastula-gastrula stage, 5 somites and 20 somites of brachydanio rerio and by the medioblastula-gastrula stage, 5 somites and 20 somites of trout at a concentration from 0.001 g/ml to 0.1 g/ml. The cultures of cells treated in vitro with embryonic extracts have been compared with the control untreated cell cultures.
3) The treated cancer cells cultures and the untreated control cultures have been submitted to the following valuations: cellular growth curve and p53 antioncogene activation. Such activation has been evaluated either by immunoistochemical methods, with an alkaline phosphatase detector, or by cytofluorometric method.

The obtained results have been the following ones: slowing of the growth curve of the cancer cells treated with the extracts and activation of the p53 antioncogene.

The activation of such antioncogene is an important index of the anti-tumour activity of the embryonic extracts.

Moreover the extracts have been used in an in vivo experimentation on mice, as will be described in a specific Example, and in numerous other pathologies as reported below.

The results obtained from the experimentation show that the embryonic extracts according to the present invention may be successfully used in the preparation of compositions suitable to the treatment of the pathologies controlled by the p53 antioncogene which, beside tumorous pathologies, include several autoimmune diseases such as the autoimmune thyroidites, the lupus erythematosus, the rheumatoid arthritis, genetic pathologies such as the pigmentous scleroderma and the psoriasis, and viral pathologies such as the sclerose en plaques, the AIDS etc.

Said compositions are formulated with pharmacologically acceptable diluents and excipients, in solid, semisolid or liquid form depending on the kind of administration and pathology.

In particular said compositions are prepared in the form of liquid solutions for parenteral, topical, oral and spray use. eye-drops. semisolid preparations such as creams, gels, ointments and pastes, solid preparations such as powders and freeze-dried products, impregnated gauzes, urethral sticks, oral emulsions and sprays, syrups, simple and coated granulates, simple and coated tablets, soft and hard, simple and modified release capsules, suppositories, vaginal globuli and suppositories and plasters.

The compositions according to the invention may be used for the human therapy of the pathologies controlled by the p53 antioncogene with a posology which provides for the administration of a quantity from 10 µg to 200 mg of embryonic extract per day.

For illustrative but not limitative aim of the invention the following Examples are reported.

### EXAMPLE 1

50,000 trout spawn at the embryonic development stage of 5 somites are taken.

The spawn are taken by mild ventral squeezing, after a light narcosis of the fish. The spawn are then rinsed many times with bidistilled water and finally washed with 60% ethyl alcohol.

Various sonication cycles are applied for about 2 hours. The weight of the mixture is about 1 kg. A solvent is prepared consisting of glycerin and 30% ethyl alcohol, in a 85:15 v/v ratio. The solvent is added to a weight of the mixture equal to 5 kg.

Then the mixture is treated with a vacuum turbodiffuser, alternating settling periods, for about 12 hours.

Subsequently it undergoes maceration keeping under stirring with a paddle-stirrer for 6 hours and it is left to decant under settling for 36 hours.

The mixture is filtered by 90 micrometers filter cartridges, then it is filtered by 10 micrometers cartridges in order to remove the spawn shells and the fat.

The solution, about 5 kg, is diluted to 50 kg with ethyl alcohol.

The solution is filtered which appears clear with 5 micrometers filter cartridges.

Such solution forms the liquid formulation for the oral administration. According to the desired dosage it may be further diluted with 30% ethyl alcohol.

The preferred concentration is 0.1 embryo/ml.

50 microliters of the so obtained extract have been inoculated in a culture of 3.5 x 10⁵ cancer cells of glioblastoma multiforme in comparison to an untreated control culture. The results have been the following ones: in the treated cancer cells occurred an activation of the p53 antioncogene 25% greater than the untreated ones.

### EXAMPLE 2

The embryonic extract prepared according to the method as in the Example 1, however consisting of trout embryos at the 20 somites development stage, has been inoculated as in the Example 1 in a culture of cancer cells of glioblastoma multiforme in comparison with a culture of cancer cells of control glioblastoma.

The results have been the following ones: in the treated cancer cells occurred an activation of the p53 antioncogene 20% greater than the untreated ones.

### EXAMPLE 3

20 mg of brachydanio rerio embryos at the medioblastula-gastrula embryonic development stage have been taken.

The taking has been carried out by the previously described method, that is the spawn have been taken at the moment of medioblastula-gastrula embryonic growth by mild ventral squeezing, after light narcosis of the fish.

The extract has been obtained acting as the method described in the Example 1.

50 microliters of the so obtained extract have been inoculated in a culture of 3.5 x 10⁵ cancer cells of glioblastoma multiforme in comparison with an untreated culture of cancer cells of glioblastoma multiforme.

The results have been the following ones: a 18% activation of the p53 antioncogene occurred 18% greater in the treated cancer cells with respect to the untreated ones.

### EXAMPLE 4 (comparison)

The extract prepared as in the Example 3, but consisting of brachydanio rerio embryos at the 5 somites development stage, has been inoculated in a culture of cancer cells of glioblastoma multiforme in comparison with a culture of untreated cancer cells of glioblastoma, as in the Example 3.

The obtained results have been the following ones: unsuccessful activation of the p53 antioncogene in the treated cancer cells as in the control ones.

### EXAMPLE 5 (comparison)

The extract prepared as in the Example 3, but consisting of brachydanio rerio embryos at the 20 somites stage, has been inoculated in untreated cultures of cancer cells of glioblastoma multiforme, as in the Example 3.

The obtained results have been the following ones: unsuccessful activation of the p53 antioncogene in the treated cancer cells as in the control ones.

### EXAMPLE 6 (comparison)

The extract prepared as in the Example 1, but consisting of trout embryos at the medioblastula-gastrula stage has been inoculated in cultures of cancer cells of glioblastoma multiforme in comparison with untreated cultures of cancer cells of glioblastoma multiforme, as in the Example 1.

The obtained results are the following ones: unsuccessful activation of the p53 antioncogene in the treated cancer cells as in the control ones.

### EXAMPLE 7 (in vivo experimentation on mice)

A million cells of the Lewis tumour, whose mechanical suspension has already been described (Sava G., Giraldi T., Lassiani L.. Dogani R. (1983) - Chem. Biol. Interaction 46-131) has been administered, inoculating it subcutaneously, to two different groups of mice, consisting each of 10 C57BL/6 mice. To a group 50 microliters of a Dulbecco phosphate buffered saline (DPS) solution have been administered as well containing 2 x 10,000 embryos of drosophila/ml at the blastoderma stage, submitted to sonication 2 times per 10 seconds, to the second group 50 microliters of DPS only.

The primary tumour growth curve and the survival times have been estimated.

The obtained results pointed out that in the group of the treated mice the cancer growth turned out to be slowed in a statistically significative manner (Student T at the 7th day of growth: 12.031, P < 0.0001; at the 9th day T = 30.977, P < 0.0001; at the 11th day T = 43.4, P < 0.0001; 15th day T = 31.151, P < 0.0001), just as the survival turned out to be increased (Student T = 20.661, P < 0.0001).

## Claims

1. Pharmaceutical composition suitable for the treatment of the tumours and the pathologies controlled by the p53 antioncogene, containing as active substance an embryonic extract taken in specific stages of the embryonic development depending on the specific tumour and the specific pathology and dependent upon the species of origin of the extract, **characterized in that** said embryonic extract is taken from the species comprising trout in stages of embryonic development of 5 and 20 somites, brachydanio rerio in stage of embryonic development of medioblastula-gastrula and drosophila in stage of embryonic development of blastoderma.

2. Composition as claimed in claim 1, **characterized in that** said tumours and said pathologies controlled by the p53 antioncogene include the primitive tumour of the liver, the glioblastoma multiforme, the ovarioncus, the Lewis tumour, the autoimmune thyroidites, the lupus erythematosus, the rheumatoid arthritis, the pigmentous scleroderma, the psoriasis, the sclerose en plaque and the AIDS.

3. Process for the preparation of compositions as defined in claim 1, comprising:
a) taking embryos at a stage specific of the embryonic development;
b) treating said embryos with several sonication cycles;
c) adding a solvent;
d) stirring the mixture obtained in the step c) by a turbodiffuser;
e) filtering the mixture first by 90 micrometers filters and then with 5-10 micrometers filters, **characterized in that** said specific stage of the embryonic development of step a) is selected from the stage of mediobastula-gastrula for brachydanio rerio, 5 somites and 20 somites for trout and blastoderma for drosophila, and said solvent of step c) consists of a mixture of glycerin and a 30% v/v ethyl alcohol aqueous solution, in a 85/15 v/v ratio.

4. Process as claimed in claim 3, **characterized in that** the ratio by weight between said solvent and said embryos ranges from 20:1 to 2:1.

5. Process as claimed in claim 3, **characterized in that** the stirring of the step d) is carried out at room temperature for a time from 2 hours to 15 hours.

6. Use of embryonic extracts as defined in claim 1 for the preparation of compositions for the treatment of the tumours and the pathologies controlled by the p53 antioncogene, in mixture with pharmacologically acceptable diluents and excipients in solid, semisolid or liquid form, for the administration by parenteral, oral or topical way.

## Patentansprüche

1. Pharmazeutische Zusammensetzung, die für die Behandlung von vom Antionkogen p53 gesteuerten Tumoren und Krankheiten geeignet ist, wobei die Zusammensetzung als Wirkstoff einen Embryonalextrakt enthält, der in Abhängigkeit vom speziellen Tumor und von der speziellen Krankheit sowie in Abhängigkeit von der Ursprungs-Art des Extrakts aus spezifischen Stadien der embryonalen Entwicklung gewonnen ist, **dadurch gekennzeichnet daß** der Embryonalextrakt aus der Gruppe von Arten gewonnen wird, die folgende umfaßt: Forelle in den Stadien der Embryonalentwicklung von 5 und 20 Somiten, Brachydano rerio im Stadium der Embryonalentwicklung der Medioblastula-Gastrula, und Drosophila im Stadium der Embryonalentwicklung des Blastoderma.

2. Zusammensetzung nach Anspruch 1, **dadurch gekennzeichnet daß** die von vom Antionkogen p53 gesteuerten Tumoren und Krankheiten den primitven Lebertumor, das multiforme Glioblastoma, den Ovarioncus, den Lewis-Tumor, die autoimmun-Thyroidites, den Lupus erythematosus, die rheumatoide Arthritis, das pigmentöse Skleroderma, Psoriase, Plaque-Sklerose und AIDS umfassen.

3. Verfahren zur Herstellung von Zusammensetzungen nach Anspruch 1, folgende Schritte umfassend:
a) die Entnahme von Embryonen in einem für die Embryonalentwicklung spezifischen Stadium,
b) die Behandlung der Embryonen in mehreren Beschallungszyklen,
c) die Zugabe eines Lösemittels,
d) die Verwirbelung der nach Schritt c) erhaltenen Mischung mit einem Turbodiffusor,
e) die Filtration der erhaltenen Mischung, zunächst mit 90-Mikrometer Filtern, dann mit 5-bis 10-Mikrometer Filtern, **dadurch gekennzeichnet daß** das spezifische Stadium der Embryonalentwicklung nach Schritt a) für Brachydano rerio aus dem Stadium der Medioblastula-Gastrula, für die Forelle aus dem 5- und 20-Somiten-Stadium und für Drosophila aus dem Blastoderma-Stadium gewählt ist, und dadurch daß das Lösemittel nach Schritt c) aus einer Mischung aus Glycerin und einer 30 Vol-%igen wässerigen alkoholischen Lösung im Volumenverhältnis 85/15 besteht.

4. Verfahren nach Anspruch 3, **dadurch gekennzeichnet, daß** das Gewichtsverhältnis zwischen dem Lösemittel und den Embryonen von 20:1 bis 1:20 beträgt.

5. Verfahren nach Anspruch 3, **dadurch gekennzeichnet, daß** die Verwirbelung nach Schritt d) bei Raumtemperatur während eines Zeitraums von 2 Stunden bis 15 Stunden durchgeführt wird.

6. Verwendung von Embryonalextrakten nach Anspruch 1, zur Herstellung von Zusammensetzungen für die Behandlung von vom Antionkogen p53 gesteuerten Tumoren und Krankheiten in Vermengung mit pharmazeutisch verträglichen Verdünnungs- und Streckmitteln in fester, halbfester oder flüssiger Form zur parenteralen, oralen oder topischen Verabreichung.

## Revendications

1. Composition pharmaceutique adaptée pour le traitement des tumeurs et des pathologies contrôlées par l'anti-oncogène p53, contenant comme substance active un extrait embryonnaire pris à des stades spécifiques du développement embryonnaire selon la tumeur spécifique et la pathologie spécifique et selon l'espèce d'origine de l'extrait, **caractérisée en ce que** ledit extrait embryonnaire est prélevé sur les espèces comprenant la truite aux stades de développement embryonnaire 5 et 20 somites, le brachydanio rerio au stade de développement embryonnaire de medioblastula-gastrula et la drosophile au stade de développement embryonnaire de blastoderme.

2. Composition selon la revendication 1 **caractérisée en ce que** lesdites tumeurs et lesdites pathologies contrôlées par l'anti-oncogène p53 incluent la tumeur primitive du foie, le glioblastome multiforme, la tumeur ovarienne, la tumeur de Lewis, les thyroidites auto-immunes, le lupus érythémateux, la polyarthrite rhumatoide, la sclérodermie pigmentée, le psoriasis, la sclérose en plaque et le SIDA.

3. Procédé pour la préparation des compositions définies dans la revendication 1 consistant à :
a) prendre des embryons à un stade spécifique du développement embryonnaire ;
b) traiter lesdits embryons pendant plusieurs cycles de sonication ;
c) ajouter un solvant ;
d) agiter le mélange obtenu à l'étape c) par un diffuseur turbo;
e) filtrer le mélange d'abord dans des filtres de 90 microns puis dans des filtres de 5-10 microns, **caractérisé en ce que** le stade de développement embryonnaire à l'étape a) est choisi parmi le stade medioblastula-gastrula pour le brachydanio rerio, les stades 5 somites et 20 somites pour la truite et le stade blastoderme pour la drosophile et **en ce que** ledit solvant de l'étape c) consiste en un mélange de glycérine et d'une solution aqueuse d'alcool éthylique à 30 % v/v, dans un rapport v/v de 85/15.

4. Procédé selon la revendication 3 **caractérisé en ce que** le rapport en poids entre ledit solvant et lesdits embryons est compris entre 20:1 et 2:1.

5. Procédé selon la revendication 3 **caractérisé en ce que** l'agitation de l'étape d) est menée à température ambiante pendant une durée de 2 heures à 15 heures.

6. Utilisation des extraits embryonnaires définis dans la revendication 1 pour la préparation de compositions pour le traitement des tumeurs et des pathologies contrôlées par l'anti-oncogène p53. mélangés avec des diluants pharmacologiquement acceptables et sous forme solide, semi-solide ou liquide, pour l'administration par voie parentérale, orale ou topique.
